# EUROPEAN PATENT APPLICATION

(11) **EP 2 692 364 A1**
(43) Date of publication of application: **05.02.2014**
(21) Application number: 12005560.3
(22) Date of filing: 31.07.2012
(51) Int. Cl.: A61L 27/12, A61L 27/36

(54) **Non-plasticized hydrophilic phosphate group containing dehydrated partially purified bone replacement material**

(71) Applicant: Geistlich Pharma AG, 6110 Wolhusen (CH)
(72) Inventor: Imhof, Cornel, 6436 Muotathal (CH); Schlösser, Lothar, 64297 Darmstadt (DE); Schäffer, Birgit, 6170 Schüpfheim (CH); Bufler, Michael, 6020 Emmenbrücke (CH)
(74) Representative: Savatier, Yves

(57) **Abstract**

The invention provides:
- A non-plasticized hydrophilic dehydrated partially purified bone replacement material of natural origin, wherein substantially all non-collagenous organic material is removed while inorganic, porous osseous structure and collagenous structure of natural bone are substantially preserved, characterized in that the bone replacement material contains 0.05 to 1.5 w/w % of a saccharide and/or a sugar alcohol, and 0.7 to 5.6 w/w % of a phosphate group which is apt to react with the calcium ions of physiological fluids to give a precursor of hydroxyapatite, this phosphate group being part of a physiologically acceptable salt,
- a process for preparing a non-plasticized hydrophilic dehydrated partially purified bone replacement material, and
- the use of 0.05 to 1.5 w/w % of a saccharide and/or a sugar alcohol to confer hydrophilic properties to a non-plasticized partially purified bone replacement material of natural origin, wherein substantially all non-collagenous organic material is removed while inorganic, porous osseous structure and collagenous structure of natural bone are substantially preserved.

## Description

### Field of the invention

The present invention relates to a non-plasticized hydrophilic dehydrated partially purified bone replacement material of natural origin having a defined isotonic solution suction capacity and containing a phosphate group apt to act as precursor of hydroxyapatite and a process for preparing that non-plasticized hydrophilic dehydrated partially purified bone replacement material.

### Background of the invention

A bone defect site may result from infection, disease, trauma, developmental malformation, malignancy, surgery or other factors. Often bone volume of a certain size needs to be generated, for instance to augment a diseased jaw. In such cases a solid graft is preferably used which can additionally be stabilized. Filling materials in particulate form are limited to small cavities as healing naturally emerges only as long as no or very limited movement occurs between a distracted segment and the surrounding bone.

Several techniques, such as the use of filling materials or grafts of natural or synthetic origin, to replace diseased or missing parts are known. Due to its natural architecture, bone is very porous. In general, bone replacement material tries to mimic the chemistry and microstructure of human bone. Certain physico-chemical parameters such as crystallinity, solubility, particle size, porosity, pore structure and pore size of the material are of pivotal importance and may greatly influence bone compatibility and bone integration. An inappropriate combination of those parameters leads to failure of bone repair. It is known that pore sizes influence cell in-growth and proliferation, vascularisation, and/or cell-adhesion processes. In view of these findings, it can be emphasized that bone replacement material of natural origin has some important advantages over synthetic materials. Although autogenous bone is widely considered to be the gold standard, its application has several shortcomings and both, autogenic and allogenic bones are available only in limited quantities.

In recent years, a great deal of attention has been focussed on the preservation of the biomechanical properties of animal bone while, in parallel, satisfying the stringent regulatory biological and chemical-physical requirements for the development of bone replacement materials of animal origin.

A commercially available bone replacement material is TUTOBONE®, which is a bovine cancellous bone block (available from Tutogen Medical). It is a dehydrated, partially purified bone transplant in which the native mechanical strength is largely maintained.

U.S. Patent No. 6,942,961 describes to a two-step method for dehydrating biological tissues for producing transplants with a preserved bone structure such as TUTOBONE®. In a first step, the tissue is partially dehydrated with an organic, water-miscible solvent. In a second step, the tissue is further dehydrated by freeze drying.

TUTOBONE®, like other commercially available dehydrated partially purified bone replacement materials of animal origin with a preserved bone structure and mechanical strength, does not provide satisfying hydrophilicity and bone integration characteristics. In order to overcome the low hydrophilicity of commercially available bone blocks of animal origin, the surgeon has to rinse the bone blocks with physiological saline during or before the surgery. Such bone blocks have to be wetted by placing them for example in an empty syringe and rinsed several times with physiological saline. This is time-consuming and not practical in a surgery or hospital setting. Besides, it cannot be guaranteed, that such treated bone blocks are completely wetted and do not contain air bubbles. Such air bubbles may prevent a rapid integration of the bone replacement material. At the air-to-blood interface, no growth or regeneration is possible. The direct structural and functional connection between the surrounding living tissue and the bone replacement material is insufficient. Blood, blood components, and the cells of the patient need to integrate the graft material rapidly after implantation.

Thus, there is a need for a dehydrated partially purified bone replacement material of animal origin with a preserved bone structure and mechanical strength that has sufficient hydrophilicity to be easily wetted and rehydrated.

U.S. Patent No. 6,293,970 and US2010/0030340 disclose a plasticized load-bearing bone graft comprising a cleaned (i.e. partially purified) non-demineralized load-bearing bone graft which is impregnated with one or more plasticizers, the plasticizers being notably saccharides such as glucose, sucrose and D-galactose, and C₂ to C₇ polyols such as glycerol, adonitol, sorbitol, ribitol, galactitol, mannitol and xylitol, and the bone graft being "any bone or piece thereof obtained from a donor, for example a human or animal and/or cadaver donor". The term "plasticized" here means that free and loosely bound waters of hydration in the bone tissue are replaced with a plasticizer without altering the orientation of the collagen fibers and the associated mineral phase, thereby preserving the structure and mechanical properties of natural bone. The plasticizer is taught to be used for stabilizing the bone graft and thus prevent fractures or microfractures developing or propagating during storage, during implantation or after implantation, and for preventing the risk of disease transmission: There is no mention of using the plasticizer as a wetting agent, the issue of hydrophilicity of the bone graft being not at all dealt with in those documents. In U.S. Patent No. 6,293,970 the plasticizer is disclosed to be used at 3 % to 30 % by w/w of bone and to allow the bone graft to be directly implanted into a patient following only a brief washing in sterile isotonic saline to remove the "plasticizer associated with the immediate surfaces of the grafts" or following an extended washing (about one hour) with isotonic saline to remove "as much plasticizer as possible" (see in particular column 8, lines 14-62 and column 12, lines 10-27). In US2010/0030340, the plasticizer is disclosed to be used in "minimal quantities", which here means the minimum amount to replace the waters of hydration in the bone tissue, using a plasticizing composition containing more than 70 % (v/v) plasticizer, with the option of either implanting the plasticized graft without rehydration, or quickly rinsing the implant in sterile isotonic saline prior to implantation (see in particular [0035] and [0126]), thereby as in US Patent No. 6293970 removing only the plasticizer associated with the external surfaces of the graft.

Generally, plasticized tissues, like mummies, are biologically not very reactive.

A plasticized bone graft wherein the closed structure of natural bone is preserved by replacing the natural water of hydration without altering the orientation of the collagen fibers and the associated mineral phase is not optimal for a bone replacement material designed to induce bone repair and regeneration. Indeed, the activity of osteoblasts can be expected to be higher when the bone specific material does not have the closed structure of natural bone (bone which in vivo under normal conditions undergoes remodeling but has no need for repair or regeneration) but a more open and disrupted structure with a high specific surface that is accessible to those bone-forming cells (See notably A.V. Taubenberg et al. 2009, Biomaterials 31: 2827-2835 and M. Kerschnitzki et al. 2011 Cells Tissues Organs 194: 119-123).

Besides, when the plasticizer replaces a natural water of hydration without altering the orientation of the collagen fibers and the associated mineral phase, it is embedded in the structure of the bone graft and is thus difficult to eliminate by washing with sterile isotonic saline prior to implantation: See U.S. Patent No. 6,293,970, column 12, lines 16-18 cited above: Only "as much plasticizer as possible" is removed after about one hour of washing with isotonic saline. The effective amount of plasticizer that remains in the plasticized partially purified material after a quick rinsing with isotonic saline is difficult to evaluate: Probably, when the plasticizer is not in excess with regard to the replaced natural water of hydration most of the plasticizer present remains in the plasticized partially purified material. In vivo the plasticizer will be only slowly washed away by physiological fluids.

Possible patient non tolerance of the bone replacement material, a foreign body introduced in his body, is always a potential problem in clinical implantation, especially if the bone replacement material contains a large amount of an additive such as a plasticizer or wetting agent which is a non physiological substance.

An objective of the present invention is to provide a hydrophilic dehydrated partially purified bone replacement material of natural origin apt to be easily wetted and hydrated, which does not have the drawbacks of the bone replacement materials of the prior art, notably those of U.S. Patent No. 6,942,961 (TUTOBONE®) and those of U.S.

Patent Nos. 6,293,970 and US2010/0030340.

That objective is attained by the invention as defined in the appended claims.

### Summary of the Invention

The present invention provides a non-plasticized hydrophilic dehydrated partially purified bone replacement material of natural origin with increased hydrophilicity and bone integration, wherein substantially all non-collagenous organic material is removed while inorganic, porous osseous structure and collagenous structure of natural bone are substantially preserved, **characterized in that** the bone replacement material contains 0.05 to 1.5 w/w % of a saccharide and/or a sugar alcohol, and 0.7 to 5.6 w/w % of a phosphate group which is apt to react with the calcium ions of physiological fluids to give a precursor of hydroxyapatite, this phosphate group being part of a physiologically acceptable salt.

The invention also provides a method to manufacture the above non-plasticized hydrophilic dehydrated partially purified bone replacement material of natural origin with increased hydrophilicity and bone integration.

The invention further provides the use of 0.05 to 1.5 w/w % of a saccharide and/or a sugar alcohol to confer hydrophilic properties to a non-plasticized partially purified bone replacement material of natural origin, wherein substantially all non-collagenous organic material is removed while inorganic, porous osseous structure and collagenous structure of natural bone are substantially preserved.

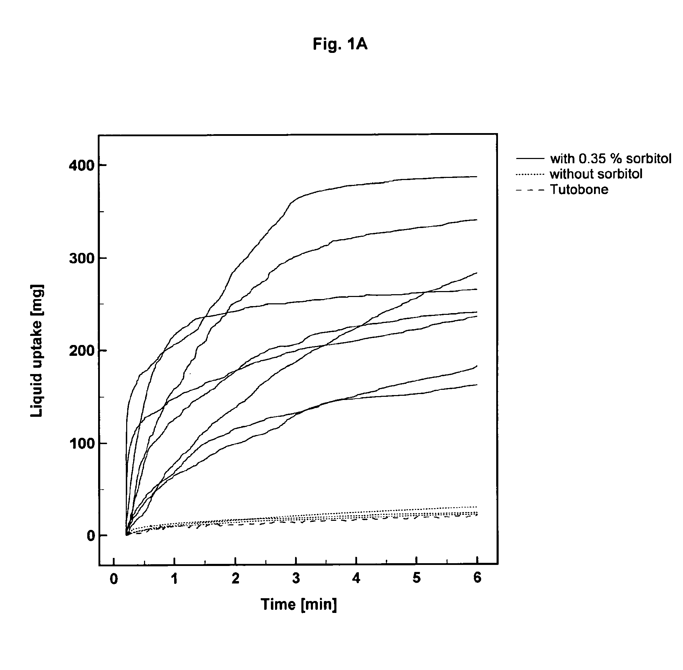

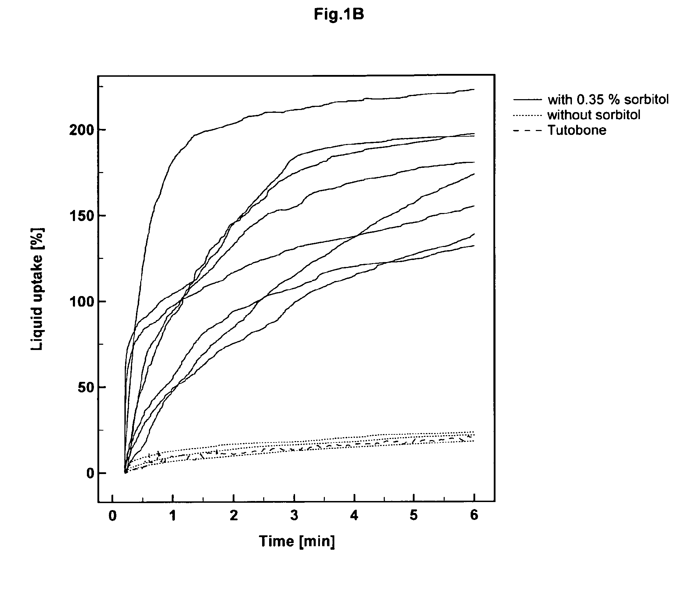

As shown by an isotonic solution suction test (see notably Example 3 and Figures 1A and 1B), the non-plasticized dehydrated partially purified bone replacement material of the invention is hydrophilic and thus apt to be immediately integrated into the surrounding tissue without the risk of forming air bubbles. Thanks to its low content of a saccharide and/or a sugar alcohol, a non-physiological substance, it will minimally perturb the functioning of the human body and thus be optimally tolerated by the patient.

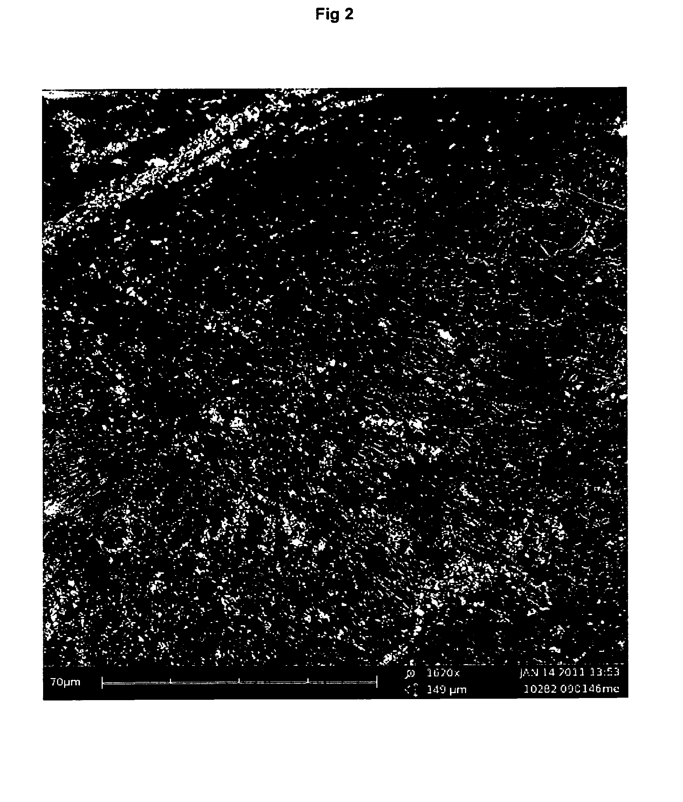

As shown by in vitro experiments of incubating a block of the non-plasticized dehydrated partially purified bone replacement material of the invention in a medium similar to physiological fluids containing calcium ions (see notably Example 4 and Figure 2), the phosphate group released reacts with the calcium ions to form a white precipitate of calcium phosphates on collagen fibrils of the block, these calcium phosphates being precursors of hydroxyapatite. This shows that in vivo, as in natural systems (see S. Gajjeraman et al. 2007 The Journal of Biological Chemistry 282, 2, pp. 1193-1204 and S. Bodhak et al. 2009 Acta Biomaterialia 5, pp. 2178-2188), the nucleation and growth of bone mineral products will take place on the collagen matrix of the hydrophilic dehydrated partially purified bone replacement material, the phosphate group playing a role in those processes.

Upon implantation in various animals, notably rats and dogs, the non-plasticized hydrophilic dehydrated partially purified bone replacement material of the invention shows excellent osteoconductive properties with no or very few adverse histopathological events (inflammatory or foreign body reactions).

### Brief description of drawings

Figure 1A shows a comparison of the isotonic solution suction capacity (liquid uptake in mg) in correlation with time for 8 different blocks of a non-plasticized hydrophilic dehydrated partially purified bone replacement material of equine origin containing 0.35 % w/w sorbitol and 1.4 % w/w of (HPO₄²⁻ and H₂PO₄⁻) according to the present invention, 3 different blocks of a non-plasticized dehydrated partially purified bone replacement material of natural origin according to the prior art, and commercially available bovine bone substitution material TUTOBONE® (Tutogen Medical GmbH).
Figure 1B shows the relative isotonic solution suction capacity (w/w ratio between the liquid uptake and the dehydrated block expressed in %) in correlation with time for 8 different blocks of a non-plasticized hydrophilic dehydrated partially purified bone replacement material of equine origin containing 0.35 % w/w sorbitol and 1.4 % w/w of (HPO₄²⁻ and H₂PO₄⁻) according to the present invention, compared to 3 different blocks of a non-plasticized dehydrated partially purified bone replacement material of natural origin according to the prior art, and commercially available bovine bone substitution material TUTOBONE® (Tutogen Medical GmbH).
Figure 2 shows a SEM (Scanning Electron Microscopy) micrograph of the surface of a block of a non-plasticized hydrophilic dehydrated partially purified bone replacement material of equine origin containing 0.35 % w/w sorbitol and 1.4 % w/w of (HPO₄²⁻ and H₂PO₄⁻) according to the present invention, after incubation in DMEM (Dulbeco's Modified Eagle's Medium) containing 10 % FCS (Fetal Calf Serum).
Figure 3 schematically illustrates a bone block in accordance with one embodiment.
Figure 4 schematically illustrates a bone pin in accordance with one embodiment.
Figure 5 schematically illustrates a bone plate in accordance with one embodiment.
Figure 6 schematically illustrates a bone tapered key in accordance with one embodiment.
Figure 7 schematically illustrates a bone bar in accordance with one embodiment.
Figure 8 schematically illustrates bone granules in accordance with one embodiment.

### Detailed description of the invention

The present invention provides a non-plasticized dehydrated partially purified bone replacement material of natural origin with increased hydrophilicity and bone integration, wherein the original bone structure and the mechanical strength of the bone are substantially preserved. In one embodiment, substantially all non-collagenous organic material is removed from the material while the inorganic, porous osseous structure and collagenous structure of natural bone are substantially preserved. The bone replacement material contains 0.05-1.5 w/w % of a saccharide and/or a sugar alcohol, and 0.7 to 5.6 w/w % of a phosphate group which is apt to react with the calcium ions of physiological fluids to give a precursor of hydroxyapatite, this phosphate group being part of a physiologically acceptable salt.

Thus, the invention relates to a non-plasticized hydrophilic dehydrated partially purified bone replacement material of natural origin, wherein substantially all non-collagenous organic material is removed while inorganic, porous osseous structure and collagenous structure of natural bone are substantially preserved, **characterized in that** the bone replacement material contains 0.05-1.5 w/w % of a saccharide and/or a sugar alcohol, and 0.7 to 5.6 w/w % of a phosphate group which is apt to react with the calcium ions of physiological fluids to give a precursor of hydroxyapatite, this phosphate group being part of a physiologically acceptable salt.

The term "non-plasticized" here means that in the hydrophilic dehydrated partially purified bone replacement material, the saccharide and/or a sugar alcohol does not replace the waters of the bone or bone-like material of natural origin with preservation of the orientation of collagen fibers and the associated mineral structure. Usually the non-plasticized hydrophilic dehydrated partially purified bone replacement material of the invention is prepared by a process comprising a step of dehydrating the partially purified bone replacement material prior to introducing the saccharide and/or a sugar alcohol, such as to cause some alteration in the orientation of the collagen fibers, resulting in a more open and disrupted collagen structure.

According to the present invention, by the term of "natural origin" is intended any bone or bone-like material that is derived from vertebrates including, but not limited to, all kinds of mammalians such as human, cow, horse, pig, etc. and any bone or bone-like material that is derived from invertebrates, such as corals. In certain embodiments, bovine, porcine or equine bone is used for the purpose of the present invention.

By the term "partially purified bone replacement material" is intended any bone or bone-like structure from which substantially all organic non-collagenous material is removed while the inorganic, porous osseous structure and the collagenous structure are substantially preserved.

The term "hydrophilic" means that the dehydrated partially purified bone replacement material is capable of being wetted by water, therefore capable of being easily rehydrated by absorbing an isotonic solution or a physiological fluid, and thus presents no risk of forming an air bubble when implanted.

The term "saccharide" notably includes hexoses such as glucose, mannose or galactose; pentoses such as ribose or arabinose; ketoses such as ribulose or fructose; disaccharides such as sucrose, lactose, or maltose, oligosaccharides like cyclodextrin. The saccharide may be present in the D- or in the L- form. Certain embodiments utilize glucose.

Sugar alcohols included in the invention are notably sorbitol, maltitol, lactitol, mannitol and xylitol. Certain embodiments utilize sorbitol.

The term "phosphate group which is apt to react with the calcium ions of physiological fluids to give a precursor of hydroxyapatite" means any phosphate group which in the human body by reaction with the calcium ions of physiological fluids, in particular blood, preferably at a pH of 6.0 to 8.0 and a temperature of 36 to 38 °C, is prone to give a precursor of hydroxyapatite Ca₁₀(PO₄)₆(OH)₂, the thermodynamically most stable and less soluble of calcium phosphate salts which is an essential component of bones.

Examples of such a phosphate group are PO₄³⁻, HPO₄²⁻ and H₂PO₄⁻.

The physiologically acceptable salt which contains the phosphate group is a phosphate salt with a physiologically acceptable cation such as e.g. Na⁺, K⁺, Ca²⁺, Mg²⁺ and Sr²⁺ which does not significantly perturb the pH balance of physiological fluids.

Examples of such a physiologically acceptable salt include: Na₃PO₄, Na₂HPO₄, K₂HPO₄, NaH₂PO₄, KH₂PO₄, Ca₃(PO₄)₂, CaHPO₄ Ca₈(HPO₄)₂(PO₄)₄, a hydrate thereof, and a mixture thereof.

In one embodiment sodium phosphate buffer is used as a physiologically acceptable salt, the phosphate groups apt to react with the calcium ions of physiological fluids apt to give a precursor of hydroxyapatite being then HPO₄²⁻ and H₂PO₄⁻.

The phosphate group contained in that material, which is apt to react with the calcium ions of physiological fluids to give a precursor of hydroxyapatite, plays an important role in promoting bone formation.

It was shown in vitro that after incubation of a block of the non-plasticized hydrophilic dehydrated partially purified bone replacement material of the invention for 72 hours in a medium similar to physiological fluids which contained calcium ions, namely DMEM (Dulbeco's Modified Eagle's Medium) containing 10 % FCS (Fetal Calf Serum), the phosphate group reacts with the calcium ions to form of a white precipitate of calcium phosphates on collagen fibrils which was visible by SEM analysis (see Fig. 2). That precipitate was shown to contain two types of calcium phosphates of different morphologies, both of which can be described as precursors of hydroxyapatite.

Those in vitro experiments (see Example 4) provide a strong indication that in vivo, as in natural systems (see S. Gajjeraman et al. 2007 The Journal of Biological Chemistry 282, 2, pp. 1193-1204 and S. Bodhak et al. 2009 Acta Biomaterialia 5, pp. 2178-2188), the nucleation and growth of bone mineral products will take place on the collagen matrix of the hydrophilic dehydrated partially purified bone replacement material, the phosphate group contained in the latter which is released in physiological fluids playing a role in such reactions. With its low content of non physiological substance, the non-plasticized hydrophilic dehydrated partially purified bone replacement material of the invention will thus when implanted be in a position to mimic natural systems.

The invention also concerns a block of a non-plasticized hydrophilic dehydrated partially purified bone replacement material as defined above, which when incubated in a medium similar to physiological fluids containing calcium ions gives rise to a precipitate of calcium phophates on the collagen matrix.

Upon implantation in various animals, notably rats and dogs, the non-plasticized hydrophilic dehydrated partially purified bone replacement material of the invention showed excellent osteoconductive properties with no or very few adverse histopathological events (inflammatory or foreign body reactions).

To be in a position to play such a role, the phosphate group should be contained in a sufficient quantity, usually at least 0.7 w/w %, preferably at least 1.4 w/w %, of the hydrophilic dehydrated partially purified bone replacement material.

When released in excess in physiological fluids the phosphate group might cause large precipitation of calcium phosphates, leading to a possible adverse inflammatory response. Such a response does not occur when the phosphate group is not higher than 5.6 w/w %, preferably 2.8 w/w % of the hydrophilic dehydrated partially purified bone replacement material.

The non-plasticized hydrophilic dehydrated partially purified bone replacement material of the invention thus usually contains 0.7 to 5.6 w/w %, preferably 1.4 to 2.8 w/w %, of a phosphate group which is apt to react with the calcium ions of physiological fluids to give a precursor of hydroxyapatite.

The invention also concerns the use of 0.7 to 5.6 w/w % of a phosphate group apt to react with the calcium ions of physiological fluids to give a precursor of hydroxyapatite, for enhancing the osteoconductive properties of a partially purified bone replacement material of natural origin, wherein substantially all non-collagenous organic material is removed while inorganic, porous osseous structure and collagenous structure of natural bone are substantially preserved, with no or very few adverse histopathological events (inflammatory or foreign body reactions).

In certain embodiments, the non-plasticized hydrophilic dehydrated partially purified bone replacement material is coated with glucose and sodium phosphate buffer.

In certain embodiments, the non-plasticized hydrophilic dehydrated partially purified bone replacement material is coated with sorbitol and sodium phosphate buffer.

In certain embodiments, the non-plasticized hydrophilic dehydrated partially purified bone replacement material further contains one or more pharmaceutically active agents.

Examples of suitable pharmaceutically active agents are taurolidine, viricides, microbicides, antibiotics, amino acids, peptides, proteins, vitamins, co-factors for protein synthesis, hormones, living cells including stem cells, enzymes, antigenic agents, antitumor agents, immunosuppressants and growth factors.

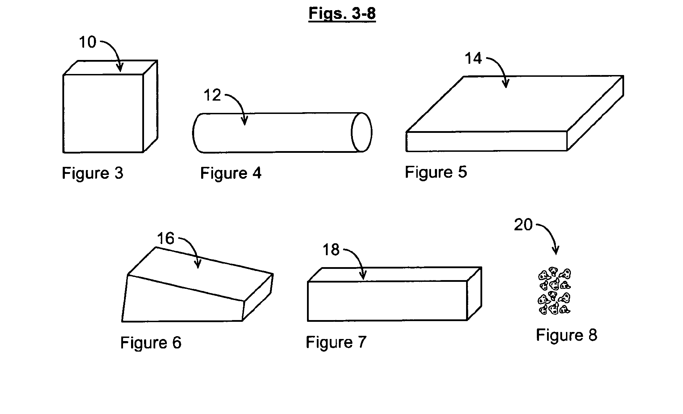

The non-plasticized dehydrated partially purified bone replacement material may have any shape including, without limitation, a block 10 (Fig. 3), a pin (bolt) 12 (Fig. 4), a plate 14 (Fig. 5), a tapered key or strip 16 (referred to as "cotter" in the U. K., Fig. 6), a bar 18 (Fig. 7), or a bone granulate 20 (Fig. 8), and the like.

In a first embodiment the structure is a block 10 as shown in Fig. 3, in a second embodiment a strip as shown in Fig.6, and in a third embodiment a granulate as shown in Fig. 8.

The invention further relates to the use of a bone replacement material as described herein as remodelling implant.

The invention also concerns the use of 0.05 to 1.5 w/w % of a saccharide and/or a sugar alcohol to confer hydrophilic properties to a non-plasticized partially purified bone replacement material of natural origin, wherein substantially all non-collagenous organic material is removed while inorganic, porous osseous structure and collagenous structure of natural bone are substantially preserved.

### Preparation of the non-plasticized hydrophilic dehydrated partially purified bone replacement material

The non-plasticized hydrophilic dehydrated partially purified bone replacement material of natural origin may be produced by a process comprising:
(a) providing a partially purified bone replacement material of natural origin by first extracting lipids with organic solvents, then treating the delipidized material with a solution containing a chaotropic agent and performing extensive washing with water,
(b) soaking the dehydrated partially purified bone replacement material of natural origin in a solution containing 0.05-0.85 w/w % of a saccharide and/or a sugar alcohol, thereby providing hydrophilicity to the partially purified bone replacement material, and 10-1000 mM of a phosphate group which is apt to react with the calcium ions of physiological fluids to give a precursor of hydroxyapatite, this phosphate group being part of a physiologically acceptable salt selected from the group consisting of Na₃PO₄, Na₂HPO₄, K₂HPO₄, NaH₂PO₄, KH₂PO₄, Ca₃(PO₄)₂, CaHPO₄ Ca₈(HPO₄)₂(PO₄)₄, a hydrate thereof, and a mixture thereof,
(c) freeze-drying the soaked hydrophilic partially purified bone replacement material, thereby performing dehydration, and
(d) sterilizing the hydrophilic dehydrated partially purified bone freeze-dried bone replacement material,
and a further intermediate dehydrating step performed on either on the delipidized bone replacement material of natural origin obtained during step (a), or the partially purified bone replacement material of natural origin obtained at the end of step (a), such as to disrupt the collagen structure and cause alteration of the orientation of the collagen fibers.

That further intermediate dehydrating step may be performed notably by vacuum-drying at a temperature above 0 °C, e.g. from 5 to 50 °C, in particular from 10 to 40 °C, or by vigorously blowing an inert gas such as nitrogen though the material. Freeze-drying is not appropriate here because fixation of bone soft tissue by freezing results in the closed structure of natural bone remaining essentially the same without any significant disruption of the collagen structure.

By taking Scanning Electron Microscopy micrographs of the material before and after that dehydrating step, it can be shown that the material presents after that step a more open, disrupted and irregular structure of collagen than before, corresponding to an alteration of the orientation collagen fibers. That disruption of the collagen structure is particularly apparent when the intermediate dehydrating step is performed on the partially purified bone replacement material of natural origin obtained at the end of step (a).

Preferably the further intermediate dehydrating step is a vacuum-drying step performed at a temperature above 0 °C on the partially purified bone replacement material of natural origin obtained at the end of step (a), such as to disrupt the collagen structure and cause alteration of the orientation of the collagen fibers.

The organic solvents used for extracting lipids in step (a) may include methanol, ethanol, ethanol, propanol, hexane, cyclohexane, acetone and/or toluene.

The chaotropic agent used in step (a) comprises, for example, urea, thiourea, guanidine HCl, guanidine thiocyanate, aminoguanidine HCl, aminoguanidine bicarbonate, guanidine carbonate, guanidine phosphate, or mixtures thereof.

In one embodiment the solution used in step (b) contains 75-600 mM, in particular 100-200 mM, sodium phosphate buffer of pH 7.0.

The invention further relates to a non-plasticized dehydrated partially purified bone replacement material prepared by the above process.

### Hydrophilicity and bone integration of the non-plasticized dehydrated Partially purified bone replacement material

Bone integration, i.e., the direct physical connection of the implant material to the surrounding living host tissue may greatly increase the success of bone repair. Hydrophilicity is a major factor influencing bone integration in the case of bone replacement materials of animal origin. The more hydrophilic the material, the better and faster is the physical connection of the implant material to the surrounding tissue.

There exists no generally accepted way to measure or quantify hydrophilicity and bone integration. An indirect measurement of bone integration is to detect the isotonic solution suction capacity of the graft.

The isotonic solution suction capacity of a dehydrated partially purified bone material is the property of that material to absorb an isotonic solution in its internal structure. That property depends on the pore structure, the wettability and the capillarity of the bone material and is a good indicator of bone integration, i.e. the direct physical connection of the implant material to the surrounding living host tissue, which plays an important role in the success of bone repair.

To measure the isotonic solution suction capacity of a block of solid bone replacement material, the following method was used: On a laboratory precision weighing balance, a defined amount of an isotonic PBS (Phosphate Buffer Saline) solution was placed in a dish. One block of a dehydrated bone replacement material of a defined weight was clamped in a height adjustable holding device beside the balance. The height of the device was then adjusted so that the bone replacement material block just touched the surface of the aqueous liquid in the dish. The height of the device was fixed so that the distance to the surface of the liquid remained the same throughout the measurement. In the block of hydrophilic bone replacement material, liquid was absorbed and the liquid uptake was detected with the balance as a function of time.

The isotonic solution suction capacity of a dehydrated partially purified bone replacement material is defined as the mass of isotonic solution absorbed. The relative isotonic suction capacity is defined as the isotonic solution suction capacity per mass unit of the dehydrated partially purified bone replacement material.

It has surprisingly been found that coating of non-plasticized dehydrated partially purified bone replacement material of natural origin with a saccharide and/or a sugar alcohol, and with a physiologically acceptable salt, so that it contains 0.05 to 1.5 w/w % of a saccharide and/or a sugar alcohol, enormously enhances the isotonic solution suction capacity.

The relative isotonic solution suction capacity of such treated bone replacement materials is at least 50 % within 5 minutes but may be at least 100 % within 5 minutes or even at least 200 % within 5 minutes.

In certain embodiments, it was found that about 50 % of the total liquid uptake is reached within less than about two minutes, or even within less than about one minute.

In certain embodiments, it was found that such a beneficial isotonic solution suction capacity can be achieved by coating the non-plasticized dehydrated partially purified bone replacement material of natural origin with a smaller amount of a saccharide and/or a sugar alcohol, so that it contains 0.05 to 0.8 w/w %, or even 0.05 to 0.5 w/w %, of a saccharide and/or a sugar alcohol.

These findings are associated with fundamental advantages, in particular:
- substantially immediate integration of the graft with the surrounding tissue;
- the surgeon does not need to pre-treat the bone replacement material prior to its use and therefore a decreased risk of contamination of the material may be obtained;
- the saccharide or sugar alcohol, which is a non physiological substance designed to be washed away by physiological fluids, is kept to a minimum, therefore the functioning of the human body is minimally disturbed and the implanted hydrophilic bone material will be optimally tolerated by the patient;
- with its low content of non physiological substance, the non-plasticized hydrophilic dehydrated partially purified bone replacement material of the invention will when implanted be in a position to mimic natural systems and allow nucleation and growth of bone mineral products on the collagen matrix of the material (see S. Gajjeraman et al. 2007 The Journal of Biological Chemistry 282, 2, pp. 1193-1204 and S. Bodhak et al. 2009 Acta Biomaterialia 5, pp. 2178-2188);
- improved and faster healing results than prior techniques.

The following examples illustrate the invention without restricting its scope.

**Example 1** Preparation of non-plasticized dehydrated partially purified blocks of spongiosa bone of equine origin containing 1.5 w/w % of glucose and 1.4 w/w % of HPO₄²⁻ and H₂PO₄⁻.

Spongiosa bone of equine origin was cut to blocks of 12 X 12 X 5 mm.

The following steps were performed on those blocks: selective washing steps with water and/or an organic solvent such as acetone or an alcohol (e.g. ethanol), virus deactivation steps using sodium hydroxide and/or hydrogen peroxide, delipidization with an organic solvent such as methanol, ethanol, propanol, hexane, cyclohexane, acetone, or toluene, and washing steps with a solution containing a chaotropic salt such as urea or guanidine hydrochloride at a temperature between 4°C to 35 °C followed. The resulting blocks were rinsed thoroughly with water, then submitted to a step of vacuum-drying at room temperature for 240 minutes at a pressure below 20'000 Pascal.

Observation of those blocks by Scanning Electron Microscopy before the step of vacuum-drying (dried by freeze-drying) and after that step showed a substantial difference in the collagen structure: After the vacuum-drying step the collagen structure was more open, disrupted and irregular, corresponding to an alteration of the orientation of collagen fibers.

Analysis of the blocks obtained after the vacuum-drying step by various techniques including semiquantitative 2D SDS-PAGE showed the presence of less than 1 % of extractable proteins other than collagen.

The blocks were then soaked in a 0.86 w/w % glucose solution containing 200mM sodium sodium phosphate buffer of pH 7.0 (prepared by dissolving NaH₂PO₄ in demineralised water and adjusting the pH with sodium hydroxide) at room temperature for 3 hours, taken out of the aqueous solution with tweezers, then freeze dried and sterilized using gamma irradiation. The obtained blocks were shown to contain about 1.5 w/w % of glucose and 1.87 w/w % of HPO₄²⁻ and H₂PO₄⁻.

**Example 2** Preparation of non-plasticized dehydrated partially purified blocks of spongiosa bone of equine origin containing 0.35 w/w % of sorbitol and 1.4 w/w % of phosphate HPO₄²⁻ and H₂PO₄⁻

Spongiosa bone of equine origin was cut to blocks of 10 X 10 X 5 mm.

The following steps were performed on those blocks: selective washing steps with water and/or an organic solvent such as acetone or an alcohol (e.g. ethanol), virus deactivation steps using sodium hydroxide and/or hydrogen peroxide, delipidization with an organic solvent such as methanol, ethanol, propanol, hexane, cyclohexane, acetone, or toluene, and washing steps with a solution containing a chaotropic salt such as urea or guanidine hydrochloride at a temperature between 4°C to 35 °C followed. The resulting blocks were rinsed thoroughly with water, then submitted to a step of vacuum-drying at room temperature for 240 minutes at a pressure below 20'000 Pascal.

Observation of those blocks by Scanning Electron Microscopy before the step of vacuum-drying (dried by freeze-drying) and after that step showed a substantial difference in the collagen structure: After the vacuum-drying step the collagen structure was more open, disrupted and irregular, corresponding to an alteration of the orientation of collagen fibers.

Analysis of the blocks obtained after the vacuum-drying step by various techniques including semiquantitative 2D SDS-PAGE showed the presence of less than 1 % of extractable proteins other than collagen.

The blocks were then soaked in a 0.2 w/w % sorbitol aqueous solution containing 150 mM sodium phosphate buffer of pH 7.0 (prepared by dissolving NaH₂PO₄ in demineralised water and adjusting the pH with sodium hydroxide) at room temperature for 2 hours, taken out of the aqueous solution with tweezers, then freeze-dried and sterilized using gamma irradiation. The obtained blocks were shown to contain about 0.35 w/w % of sorbitol and 1.4 w/w % of HPO₄²⁻ and H₂PO₄⁻.

**Example 3** Measurement of isotonic solution suction capacity in correlation with time for non-plasticized dehydrated partially purified bone materials according to the invention

To measure the isotonic solution suction capacity of a block of solid bone replacement material, the following method was used:
On a laboratory precision weighing balance, a defined amount of an isotonic PBS solution was placed in a dish. One block of a dehydrated bone replacement material of a defined weight was clamped in a height adjustable holding device beside the balance. The height of the device was then adjusted so that the bone replacement material block just touched the surface of the aqueous liquid in the dish. The height of the device was fixed so that the distance to the surface of the liquid remained the same throughout the measurement. In the block of hydrophilic bone replacement material, liquid was absorbed and the liquid uptake was detected with the balance as a function of time.

The above method was used for measuring the isotonic solution absorption capacity of:
- 8 different blocks (having a density between 0.230 and 0.500 g/cm³) of a non-plasticized hydrophilic dehydrated partially purified bone replacement material of equine origin according to the invention, containing 0.35 w/w % of sorbitol and 1.4 w/w % of HPO₄²⁻ and H₂PO₄⁻ prepared as described in Example 2
- 3 different blocks of a non-plasticized dehydrated partially purified bone replacement material of equine origin prepared as described in Example 2 second paragraph (without sorbitol or phosphate), and
- commercially available block of bovine substitution material TUTOBONE® (Tutogen Medical GmbH).

The results are represented in Figures 1A and 1B.
Figure 1A shows the isotonic solution suction capacity (liquid uptake in mg) in correlation with time for the above blocks.
Figure 1B shows the relative isotonic solution suction capacity (w/w ratio between the liquid uptake and the dehydrated block, expressed in %) in correlation with time for the above blocks.

As apparent from Figure 1A:
- the presence of 0.35 % sorbitol and 1.4 w/w % of HPO₄²⁻ and H₂PO₄⁻dramatically increases the isotonic solution suction capacity,
- more than 50 % of the total liquid uptake is reached within less than 2 minutes, or even less than 1 minute .

As apparent from Figure 1 B:
- the relative isotonic solution suction capacity with 5 minutes is at least 100 % for all blocks,
- but may be at least 150 % (5 blocks) or even at least 200 % (one block).

The above experiments show the high hydrophilicity of the blocks of non-plasticized dehydrated partially purified bone materials according to the invention containing 0.35 w/w % of sorbitol and 1.4 w/w % of phosphate HPO₄²⁻ and H₂PO₄⁻.

In a series of similar experiments it was shown that blocks of non-plasticized dehydrated partially purified bone materials according to the invention containing 0.05, 0.2, 0.46, 1.14 or 1.50 w/w % of sorbitol and 1.4 w/w % of phosphate HPO₄²⁻ and H₂PO₄⁻ have the same high hydrophilicity (the measured relative isotonic solution absorption capacity was not significantly different for those various sorbitol contents).

**Example 4** Formation by reaction with calcium ions present in a medium similar to physiological fluids of a precipitate likely to be a precursor of hydroxyapatite on the surface of non-plasticized dehydrated partially purified bone materials according to the invention.
- A block of about 150 mg of a non-plasticized hydrophilic dehydrated partially purified bone replacement material of equine origin according to the invention, containing 0.35 w/w % of sorbitol and 1.4 w/w % of HPO₄²⁻and H₂PO₄⁻ prepared as described in Example 2, and
- 3 ml of 150 mM sodium phosphate buffer (containing the same amount of HPO₄²⁻ and H₂PO₄⁻ as the above block ) without any block,
were incubated for 72 hours at 37 °C in 3 ml of in DMEM (Dulbeco's Modified Eagle's Medium) containing 10 % FCS (Fetal Calf Serum) (a medium similar to physiological fluids containing a concentration of calcium ions of about 65 mg/l).

The medium obtained by incubating the sodium phosphate buffer became and stayed turbid during all the incubation time whereas the medium obtained by incubating each the above block stayed clear during all the incubation time.

The above block was taken out of the incubation medium, fixed with 4% PFA (paraformaldehyde), dried and analysed by SEM (Scanning Electron Microscopy).

See Figure 2, which represents a micrograph of that block: The precipitate is depicted in white on the surface of the material, the collagen fibrils being visible as a greyish network underneath the precipitate.

To further characterize the reaction of the bloc with calcium ions present in the medium the Ca concentration of the medium was measured prior to and after 24h incubation with the bloc. The amount of calcium ions in solution after 24 hours incubation was measured to be much lower (about 2.0 mg/ml) than the initial amount of calcium ions (about 50 mg/ml), demonstrating reaction of the calcium ions with the phosphate groups

Since it was not possible to remove the precipitate from those blocks to analyse its composition, the precipitate formed from the turbid medium under similar conditions by incubating 3 ml of 150 mM sodium phosphate buffer without any block was washed, dried and analyzed via Scanning Electron Microscopy (REM) and Energy Dispersive X ray Spectroscopy (EDX). It was found that the precipitate contained calcium, phosphate and oxygen and included two types of calcium phosphates of different morphologies, one crystalline (sharp edged lamellas) with a molar ratio of calcium:phosphate 1:1 and the other cryptocrystalline or amorphous (fine particles agglomerates) with a ratio of 4:3. Both structural types represent a Ca:P molar ratio unequal to hydroxyapatite, which indicates that both phases can be described as precursors of hydroxyapatite. Indeed all calcium orthophosphate based salts should be transformed into hydroxyapatite, the thermodynamically most stable form of all calcium ortho-phosphate salts in an aqueous medium of pH of about 7 at a temperature of about 37°C, normal conditions found within the human body.

Those experiments show that in the hydrophilic dehydrated partially purified bone replacement material according to the invention, which contains a substantially lower amount of wetting agent than known hydrophilic bone replacement materials of the prior art, the phosphate groups HPO₄²⁻ and H₂PO₄⁻ react with the calcium ions of body fluids to form a precipitate on the surface of the biomaterial, this precipitate having the features of a precursor of hydroxyapatite.

The above in vitro experiments provide a strong indication that in vivo, as in natural systems, the nucleation and growth of bone mineral products will take place on the collagen matrix, the phosphate groups released in physiological fluids playing a role in such reactions.

## Claims

1. A non-plasticized hydrophilic dehydrated partially purified bone replacement material of natural origin, wherein substantially all non-collagenous organic material is removed while inorganic, porous osseous structure and collagenous structure of natural bone are substantially preserved, **characterized in that** the bone replacement material contains 0.05 to 1.5 w/w % of a saccharide and/or a sugar alcohol, 0.7 to 5.6 w/w % of a phosphate group which is apt to react with the calcium ions of physiological fluids to give a precursor of hydroxyapatite, this phosphate group being part of a physiologically acceptable salt.

2. A non-plasticized hydrophilic dehydrated partially purified bone replacement material according to claim 1, which contains 0.05 to 0.8 w/w % of a saccharide and/or a sugar alcohol.

3. A non-plasticized hydrophilic dehydrated partially purified bone replacement material according to any of claims 1 to 2, wherein the saccharide is glucose.

4. A non-plasticized hydrophilic dehydrated partially purified bone replacement material according to according to any of claims 1 to 2, wherein the sugar alcohol is sorbitol.

5. A non-plasticized hydrophilic dehydrated partially purified bone replacement material according to any of claims 1 to 4, wherein the phosphate group apt to react with the calcium ions of physiological fluids is selected from the group consisting of phosphate PO₄³⁻, HPO₄²⁻ and H₂PO₄⁻.

6. A non-plasticized hydrophilic dehydrated partially purified bone replacement material according to claim 1, wherein the physiologically acceptable salt is selected from the group consisting of Na₃PO₄, Na₂HPO₄, K₂HPO₄, NaH₂PO₄, KH₂PO₄, Ca₃(PO₄)₂, CaHPO₄ Ca₈(HPO₄)₂(PO₄)₄, a hydrate thereof, and a mixture thereof.

7. A non-plasticized hydrophilic dehydrated partially purified bone replacement material according to claim 6, wherein the physiologically acceptable salt is sodium phosphate buffer salt.

8. A non-plasticized hydrophilic dehydrated partially purified bone replacement material according to claim 1, **characterized in that** it contains sorbitol and sodium phosphate buffer salt.

9. A non-plasticized hydrophilic dehydrated partially purified bone replacement material according to any of the preceding claims, **characterized in that** it is of bovine, porcine or equine origin.

10. A non-plasticized hydrophilic dehydrated partially purified bone replacement material according to any of the preceding claims, **characterized in that** it further contains a pharmaceutically active agent.

11. A block of a non-plasticized hydrophilic dehydrated partially purified bone replacement material according to any of claims 1 to 10, which when incubated in a medium similar to physiological fluids containing calcium ions gives rise to a precipitate of calcium phophates on the collagen matrix.

12. A process for preparing a non-plasticized hydrophilic dehydrated partially purified bone replacement material according to any of claims 1 to 11 comprising:
(a) providing a partially purified bone replacement material of natural origin by first extracting lipids with organic solvents, then treating the delipidized material with a solution containing a chaotropic agent and performing extensive washing with water,
(b) soaking the dehydrated partially purified bone replacement material of natural origin in a solution containing 0.05-0.85 w/w % of a saccharide and/or a sugar alcohol, thereby providing hydrophilicity to the partially purified bone replacement material, and 10-1000 mM of a phosphate group which is apt to react with the calcium ions of physiological fluids to give a precursor of hydroxyapatite, this phosphate group being part of a physiologically acceptable salt selected from the group consisting of Na₃PO₄, Na₂HPO₄, K₂HPO₄, NaH₂PO₄, KH₂PO₄, Ca₃(PO₄)₂, CaHPO₄ Ca₈(HPO₄)₂(PO₄)₄, a hydrate thereof, and a mixture thereof,
(c) freeze-drying the soaked hydrophilic partially purified bone replacement material, thereby performing dehydration, and
(d) sterilizing the hydrophilic dehydrated partially purified bone freeze-dried bone replacement material,
and a further intermediate dehydrating step performed on either on the delipidized bone replacement material of natural origin obtained during step (a), or the partially purified bone replacement material of natural origin obtained at the end of step (a), such as to disrupt the collagen structure and cause alteration of the orientation of the collagen fibers.

13. A process according to claim 12, wherein the solution used in step (c) contains 75-600 mM, preferably 100-200 mM sodium phosphate buffer of pH 7.0.

14. A process according to claim 12, wherein the further intermediate dehydrating step is a vacuum-drying step performed at a temperature above 0 °C on the partially purified bone replacement material of natural origin obtained at the end of step (a), such as to disrupt the collagen structure and cause alteration of the orientation of the collagen fibers.

15. Use of 0.05 to 1.5 w/w % of a saccharide and/or a sugar alcohol to confer hydrophilic properties to a non-plasticized partially purified bone replacement material of natural origin, wherein substantially all non-collagenous organic material is removed while inorganic, porous osseous structure and collagenous structure of natural bone are substantially preserved.
